Europäisches Patentamt

**European Patent Office**

**Office européen des brevets**

(19)

(11) Publication number: **0 212 875**
**A2**

## (12) EUROPEAN PATENT APPLICATION

(21) Application number: **86305812.9**

(22) Date of filing: **29.07.86**

(51) Int. Cl.⁴: **A 61 K 9/66, A 61 K 9/10**

(30) Priority: **12.08.85 US 764933**
**30.12.85 US 814414**

(43) Date of publication of application: **04.03.87**
**Bulletin 87/10**

(84) Designated Contracting States: **AT BE CH DE FR GB IT LI LU NL SE**

(71) Applicant: **Tucker, William Gough, 3230 S, Pennsylvania Avenue, Lansing Michigan 48910 (US)**

(72) Inventor: **Tucker, William Gough, 3230 S, Pennsylvania Avenue, Lansing Michigan 48910 (US)**

(74) Representative: **McCall, John Douglas et al, W.P. THOMPSON & CO. Coopers Building Church Street, Liverpool L1 3AB (GB)**

(54) Medicinal composition and method of making same.

(57) There is disclosed a medicinal composition comprising lipid, non-lipid liquid and pharmaceutical ingredients, characterized by the fact that the ingredients are emulsified and encapsulated in a gelatine capsule as a liquid and in a physical form capable of delivering the pharmaceutical in its unaltered original state to the brush layer of the small intestine; the non-lipid ingredient being water, a water substitute not attacked by lipid enzyme activity or a mixture of water substitute and water; and the total water in the emulsion is not more than 20%; the physical form of the ingredients is as follows:

(a) the lipid ingredient is in the form of individual globules predominantly of a size less than about 3 microns in diameter;

(b) each of the globules contains the pharmaceutical ingredient dispersed therein; and

(c) each of the globules is enveloped within a surrounding layer of said non-lipid liquid ingredient adhered to the globule by surface tension, the surrounding layer serving to maintain the globules in independent form and to present only the non-lipid liquid surface to the stomach fluids.

There is also disclosed a method of making same.

## MEDICINAL COMPOSITION AND METHOD OF MAKING SAME

### BACKGROUND OF THE INVENTION

In my original application (European Publication No. 0073006, filed August 17, 1982) there are disclosed certain medicinal compositions capable of orally delivering lipid-soluble pharmaceuticals and drugs and certain methods of making such compositions.

The invention, as earlier disclosed by me, proposes a drug delivery system whereby a pharmaceutical or drug is dissolved in lipid droplets of extremely minute size (predominantly less than about 3 microns in diameter and preferably about 1 micron or less in size). Each droplet is enveloped in a water layer for passage through the stomach in unaltered form to the brush layer of the small intestine for absorption directly into the thoracic duct and hence into the blood system. Encapsulation of the emulsion of the original application required mixing the emulsion with a powder and then placing the mixture in a capsule.

The earlier disclosed method of making the delivery system includes the steps of dissolving the drug in a lipid and then emulsifying the lipid in water (generally about 30% of the total emulsion) and in the presence of a surfactant to form an emulsion in which the extremely fine lipid globules are each encased within an enveloping layer of water. The enveloping layer of water serves to keep the lipid globules in their finely-divided form, and it also serves to mask the lipid from the digestive juices of the digestive system so that the lipid globules pass unaltered through the stomach into the small bowel for transport into the bloodstream. The method also requires encapsulation with a powder.

## BRIEF DESCRIPTION OF THE PRESENT INVENTION 0212875

It has now been determined that drug-containing emulsions similar to those disclosed in my original application can be encapsulated in either soft or hard gelatin capsules without the use of the heretofore necessary powder. Such emulsions capable of direct encapsulation contain a non-lipid liquid which is not attacked by liquid enzymatic activity as the medium in which the drug-containing lipid is dispersed and which forms the enveloping layer protecting the drug from the digestive juices of the stomach, thereby ensuring the passage of the unaltered lipid globules to the brush layer of the small intestine.

The non-lipid liquid can be water, a water substitute or a mixture of water substitute and water, so long as the total water content of the emulsion is not more than about 20%, by weight. The preferred water substitute is polyethylene glycol, and it is not subject to the 20% limitation.

The emulsions of the present invention comprise a lipid ingredient, a drug dispersed in the lipid, the non-lipid liquid and one or more surfactants. Although the composition can be formed and the drugs delivered by the use of a single surfactant, it is generally preferred in the present invention that a plurality of surfactants be utilized, such surfactants being primarily lipophilic, although they also are at least partially hydrophilic.

Also, the preferred surfactants are those having either no charge (non-ionic) or an electrical charge opposite to the charge of the pharmaceutical. Thus, where the drug is cationic, anionic surfactants are preferred. Conversely, where the drug is anionic, cationic surfactants are preferred. Of course, non-ionic surfactants also may be utilized, particularly where the drug bears no appreciable electrical charge. It has been found that a reduction in the net electrical charge in the emulsion ingredients results in a more stable emulsion of longer shelf life.

One specific method of the present invention for making an emulsion of oil containing a drug which is not lipid-soluble and which bears no appreciable electrical charge involves mixing the drug with one or more non-ionic surfactants, thereby dispersing (i.e., either suspending or dissolving) the drug in the surfactants, then mixing the drug-surfactants mixture with a lipid to suspend the drug in the lipid preparatory to forming the emulsion. Another acceptable method involves mixing the lipid-insoluble drug with the lipid, then adding the surfactants to aid in dispersing the drug in the lipid. Alternatively, a water-soluble drug can be dissolved in a minor amount of water, and the water-drug solution is then mixed with a lipid in the presence of one or more non-ionic surfactants to suspend the solution in the oil. Where the drug is lipid-soluble, it is merely mixed with the lipid for solution, then emulsified.

Any one of these forms of drug-containing lipids is then mixed with the lipid-incompatible, non-lipid liquid preparatory to forming the emulsion. The lipid is then emulsified in the incompatible, non-lipid liquid to form the lipid into globules of less than about 3 microns in size and preferably of about 1 micron or less in size. In the final emulsion, each drug-containing lipid globule is surrounded or encased by an enveloping layer of the incompatible, non-lipid liquid in the final emulsion.

Non-lipid liquids of the present invention can be any liquid capable of emulsification with the drug-containing lipid, capable of forming the globule-encasing exterior layer, and capable of passing through the digestive system without being acted upon by the digestive system. Water is one of the presently preferred non-lipid liquids. Polyethylene glycol is the presently-preferred non-aqueous non-lipid liquid, since it is capable of forming an encasing layer and the digestive system recognizes it as water, does not act upon it, and permits the polyethylene glycol-covered lipid globules to pass unaltered to the small bowel. Polyethylene glycol and water mixtures act in the same manner. Additionally, polyethylene glycol, even when mixed with water, appears to adhere to the lipid globules more tenaciously than does water alone, probably due to the fact that its surface tension is normally greater than that of water alone. Preferably, any mixtures of polyethylene glycol and water can be utilized in the present invention for encapsulation in soft capsules so long as the total emulsion contains about 20% or less water, and for encapsulation in hard capsules, so long as the total emulsion contains no more than about 10% water.

As a specific example, it has been found that insulin can be successfully orally delivered by dispersing the insulin in a lipid with the aid of a mixture of surfactants, either by (1) dissolving the insulin in a mixture of non-ionic surfactants, and then utilizing the surfactant-insulin solution to place the insulin in a lipid, or (2) mixing the lipid with the insulin, then adding the surfactants to disperse the insulin in the lipid. In either event, the lipid is then emulsified with polyethylene glycol, either alone or with some water present. The resultant emulsion thus contains the insulin dispersed in the lipid, the oil being in the form of globules of a size less than about 3 microns and preferably less than about 1 micron, and the globules each being encased in a surrounding layer of polyethylene glycol or polyethylene glycol plus water. Such an emulsion passes through the digestive system without alteration, and the insulin can be delivered through the brush layer of the small intestine. Generally, the finer the emulsion the better. Emulsion of globules in the order of 0.3 micron are most preferred.

## BRIEF DESCRIPTION OF THE DRAWINGS

Figure 1 is a computer-generated graph illustrating the blood sugar levels in a dog at specific times following oral administration of insulin in accordance with the present invention.

Figure 2 is a graph similar to Figure 1 illustrating the same data with respect to a second dog.

Figure 3 is a view similar to Figures 1 and 2 illustrating similar data in connection with a third dog.

Figure 4 is a graph similar to Figure 1 illustrating the combined blood sugar levels of two different dogs at specific times following oral administration of insulin in accordance with the present invention.

Figure 5 is a graph similar to Figure 4 showing the blood sugar levels in the individual dogs from the fifth hour after insulin administration.

Figure 6 is a graph similar to Figure 4, but three weeks after administration of oral insulin.

Figure 7 is a computer-generated graph illustrating the serum levels of oral propanolol HCl in a human subject at specific times following oral administration of propanolol in accordance with the present invention.

## DETAILED DESCRIPTION OF THE INVENTION

The emulsion of this invention comprises oil or lipid droplets or globules of a size predominantly less than about 3 microns in diameter and preferably of micellar size ranging from about 0.3 to about 1.0 microns, in which the administered drug is dispersed. It has been determined experimentally that the techniques here proposed result in an emulsion which contains droplets or globules of a size less than 3.0 micron and, preferably, predominantly (on the order of 70% to 90%) droplets or globules of a size corresponding to 1.0 microns or less.

-6-    0212875

Each minute droplet or globule is enclosed or encased in a surrounding layer of non-lipid liquid, preferably water, polyethylene glycol or mixtures of polyethylene glycol and water, which is not attacked by the digestive system.

While the exact delivery mechanism is not known at this time, it appears that non-lipid liquid-encased globules of this size do not excite or trigger the enzyme bile action of the body.  The digestive system, apparently, recognizes the outer liquid layer as water and does not attack the oil globules encased in the outer layer.  Thus, oil globules of the fine micellar size contemplated by this invention can be delivered without change from the stomach and can be directly absorbed at the brush layer of the small intestine for passage directly into the thoracic duct and thence directly into the blood system for systemic circulation.  The smaller globule sizes are preferred, since the smaller the size, the less interference with the digestive system and the better final transmission into the bloodstream.

The non-lipid liquid layer can be observed microscopically, and it serves (a) to maintain the integrity of the individual oil globules; (b) to prevent agglomeration of the oil globules into a single mass, once the emulsion has been formed; and (c) to mask the oil globules from the enzyme bile action of the body.  The external layer is formed and retained by the surface tension of the non-lipid liquid component of the emulsion, and persists as the emulsion passes through the stomach and is presented to the brush layer of the intestine.  While the absorption mechanism at the brush layer is not fully known at this time, there is reason to believe that the external layer is stripped away either immediately prior to or during passage of the globule through the inter-cellular space of the brush layer itself.

The present invention is adaptable to those drugs hereinafter listed, whether lipid-soluble, or wholly or partially lipid-insoluble. It is particularly adapted to the oral delivery of pharmaceuticals or drugs which are normally parentally delivered only to the body. Typical of such drugs is insulin, which is delivered by subcutaneous injection at the present time. Other types of drugs fitting this description are doxorubicin, calcitonin, and similar drugs. The necessity of parental dosage of insulin, for example, arises from the alteration or destruction of the drug by the stomach digestive juices. Of course, the advantage of delivering such drugs by oral ingestion will be readily appreciated, particularly by those suffering from juvenile diabetes who must give themselves at least two injections of insulin per day every day of their lives. The present invention also makes possible the delivery of small dose (on the order of 1 to 5 units of insulin), thus making insulin therapy available to adult diabetes patients. The oral delivery of insulin should result in more frequent administration for better and more constant control of blood sugar levels in all diabetes patients.

The pharmaceutical component of the emulsion either (a) is dissolved directly and in the desired dosage in the initial lipid solvent component; or (b) is dispersed in the desired dosage in the lipid component. Exemplary of lipid-soluble drugs which may be utilized in this invention include Vitamin A palmitate, other retinyl esters, procarbazine, BCNU (bis-chloroethyl nitrosourea), CCNU (cis-chloroethyl nitrosourea), methyl CCNU, ascorbyl palmitate, tersterone cypionate, testosterone enanthate, nandrolone phenpropionate, cholecalciferol, chloral hydrate, pancuronium bromide, acetophenazine maleate, ambenonium chloride, anileridine hydrochloride, betamethasone sodium phosphate, bromodiphenhydramine hydrochloride, butabarbital sodium, carphenzine maleate, cephaloridine, chloral betaine, chlortetracycline hydrochloride, dehydrocholic acid, dexbrompheniramine maleate, dexrhlorpheniramine maleate, dimethyl tubocurarine iodide, diphenadione, dromostanolone propionate, ephedrine hydrochloride, erythromycin estolate, estradiol, estradiol benzoate, glycopyrrolate, hydroxocobalimin, hydroxyzine hydrochloride, hyoscyamine hydrobomide, indomethacin, isobucaine hydrochloride, isoproterenol sulfate, menadione sodium bisulfite, mephentermine sulfate, methacycline hydrochloride, methantheline bromide, methapyriline hydrochloride, methdilazine hydrochloride, methenamine, methiodal sodium, methylbenzethonium chloride, methylergonovine maleate, oxymorphone hydrochloride, paramethasone acetate, pargyline hydrochloride, paromomycin sulfate, pentazocine hydrochloride, pentolinium tartrate, perphenazine, phenazopyridine hydrochloride, phenolphthalein, phenylpropanololamine hydrochloride, poldine methylsulfate, potassium sorbate, prilocaine hydrochloride, propiomazine hydrochloride, propoxycaine hydrochloride, psuedo-ephedrine hydrochloride, pyrilamine maleate, pyrrobutamine phosphate, pyrrocaine hydrochloride, rotoxamine tartrate, sennosides A and B, simethicone, stibophen, sulfoxone sodium, testosterone, thiethylperazine maleate, triethylperazine maleate, triamcinolone diacetate, trifluoperazine hydrochloride, trimethobenzamide hydrochloride, triprolidine hydrochloride, tromethamine, warfarin potassium, amodiaquine hydrochloride, candicidin, carphenazine maleate, clindamycin phosphate, floxuridine, isoproterenol sulfate, levallorphan tartrate, menadione sodium bisulfite, methylprednisolone sodium succinate, methysergide maleate, piperazine phosphate, sodium propionate, sulfisoxazole diolamine, tetrahydrozoline hydrochloride, various prostaglandins for human or animal use, 7 alpha 19 nor testosterone, and testosterone proprionate.

Exemplary of drugs which can be suspendable or dispersible in lipids, and some of which have, at best, some limited degree of lipid solubility, are the following: valprioc acid, divalproex sodium, methylclothiazide and deserpidine, pargyline hydrochloride, deserpidine, paramethadione, ethotoin, phenacemide, ethchlorvynol, hydroflumethiazide, griseofulvin ultramicrosize, propranolol hydrochloride, primidone, dipryidamole, mesoridazine besylate, chlorthalidone, allopurinol, ibuprofen, hydroflumethiazide, carmustine, mitotane, megestrol acetate, etoposide, methyltestosterone buccal, fluoxymesterone, erythrityl tetranitrate, azathioprine, digoxin, busulfan, acyclovir sodium, hydralazine hydrocholoride, aminoglutethimide, hydrochlorothiazade USP, maprotiline hydrochloride, desoxycorticosterone acetate, reserpine USP, phenylbutazone USP, baclofen, oxyphenbutazone USP, carbamazepine USP, iodoquinol, furosemide, methyltestosterone, cyclandelate, trimipramine maleate, ethionamide, cyclothiazide, digitoxin, acetohexamide, diltiazem HCI, pimozide, estradiol, methyldopa MSD, probenecid MSD, dexamethasone MSD, metyrosine MSD, chlorothiazide sodium MSD, diflunisal MSD, ethacrynic acid MSD, hydrochlorothiazide MSD, indomethacin MSD, amiloride HCI MSD, carbidopa-levodopa MSD, mepenzolate bromide USP, probucol, trichlormethiazide USP, chlorotrianisene USP, metronidazole, griseofluvin microsize, methsuximide, prazepam, phenytoin, quinestrol, phensuximide, phenelzine sulfate, mefenamic acid, pyrvinium pamoate, ethosuximide, doxepin, metolazone, chlorpropamide, piroxicam, nifedipine, polythiazide, flucytosine, sulfamethoxazole, phenazopyridine hydrochloride, bumetanide, sulfisoxazole, levodopa, procarbazine hydrochloride, diazepam, glipizide, ergoloid mesylates, thioridazine, nortriptyline, bromocriptine mesylate, temazepam, pindolol, ethinyl estradiol, ultramicrosize griseofulvin, trichlomethiazide, methyltestosterone, halazempam, fluphenazine

0212875

hydrochloride, diazoxide, spironolacetone, oxadrolone, disopyramide phosphate, prochloperazine, trimterene, cimetidine, chlorpromazine, captopril, nadolol, hydroxyurea, bendroflumethiazide, chloral hydrate, fluphenazine hydrochloride, fluphenazine decanoate, testolacetone, simethecone, tamoxifen citrate, atenolol, oxymetholone, norethindrone and ethinyl estrodiol, phenylbutazone, glutethimide, chlorthalidone, indapamide, triazolam, fluoxymesterone, minixodil, ibuprofen, tolazamide, methylchlothiazide, danazol, chlormezanone, stanolzolol, lorazepam, guanabenz acetate, insulin and calcitonin.

Such drugs, if they are lipid-soluble, are merely incorporated into the emulsion of the present invention by dissolving them in the lipid component. However, most of these drugs are either lipid-insoluble or are not sufficiently lipid-soluble to permit a sufficient dosage level by mere dissolution in the lipid component.

These relatively lipid-insoluble drugs are preferably incorporated into the emulsion by initially dissolving or suspending the drug in either the oil or in a surfactant or, preferably, a mixture of surfactants.

One type of surfactant or emulsifier useful in the present invention are the natural gums, such as gum arabic, gum tragacanth, or the like. In the use of gum tragacanth, larger amounts of emulsion water are required, on the order of ten times the amount required for the use of gum arabic. Gum arabic normally includes an enzyme which preferably is destroyed by heating the gum to a temperature of about 80°C prior to its use in the present invention. Other natural or synthetic surfactants can also be utilized, preferably those which, although they have a minimal tendency to crystallize, may cause some problems with the retention of the water layer enveloping the oil globules. For these reasons, gum arabic is the presently most preferred surfactant, and natural gum-type surfactants are preferred.

Typical of such other surfactants or emulsifiers, both natural and synthetic, which may be utilized are the following: bentonite, sodium stearate, monovalent soaps (castile, soft soap) triethanolamine stearate, triethanolamine oleate, tweens (polysorbates), acacia, agar, alginic acid, carboxymethylcellulose sodium, carrageenan, powdered cellulose, gelatin, cetyl sulfate Na/cholesterol, lactylates (carboxylic acids) sulfated monoglycerides, spans (sorbates), sodium lauryl sulfate, calcium stearate, polyethylene glycol 400 monostearate, polyoxyl 40 stearate, (Myrj[R] 52), glycerol monostearate, hydroxyethyl cellulose, hydropropyl cellulose, hydropropyl methylcellulose, methlcellulose, povidone, propylene glycol monostearate, stearyl alcohol, and xanthan gum.

The preferred surfactants utilized in the present invention are those that are non-ionic and that are acceptable and approved for pharmaceutical use. Exemplary of such non-ionic surfactants are Tween 80, Tween 20 and Span 80. Where the pharmaceutical bears an appreciable electrical charge, the preferred surfactants are those which bear the opposite electrical charge.

Tween 80, Tween 20 and Span 80 are all commercially available non-ionic surface active agents which are complex esters and ester-ethers. The Spans are partial esters of the common fatty acids (such as lauric, palmitic, stearic and oleic), and hexitol anhydrides (hexitans and hexides) derived from sorbitol. The Tweens are derived from the Span products by adding polyoxyethylene chains to the non-esterified hydroxyls. Span 80, Tween 80 and Tween 20 are all liquids which are soluble in oils, such as corn oils, and are dispersible in water. They are both hydrophilic and lipophilic, since their free hydroxyl and oxyethylene groups are hydrophilic and their long-chain fatty acid components are lipophilic. The Spans and the Tweens are both accepted and approved as pharamceutical components.

0212875

Of course, a single surfactant can be utilized to incorporate the pharmaceutical or drug into the oil. However, I have found that mixtures of two or more surfactants are useful to lend stability to the final emulsion. The reason for this phenomenon is not completely understood at the present time, but it appears that the utilization of two or three surfactants in combination both disperses the drug in the oil and aids in forming the final emulsion. Of course, the surfactants serve both of these functions, namely (1) as an aid for incorporating the drug into the oil and (2) in obtaining an extremely fine emulsion of extended shelf life.

After the drug-oil dispersion is formed, the other emulsion ingredients are added. These emulsion ingredients may include any non-lipid liquid which is (1) capable of forming an emulsion with the lipid, (2) capable of forming an external layer on the lipid globule, and (3) capable of passage through the digestive system in unaltered form. This non-lipid component masks the lipid component and the pharmaceutical contained therein from the digestive system, since the digestive system simply recognizes the non-lipid oil as water.

While any suitable non-lipid liquid can be utilized, I presently prefer to utilize water or polyethylene glycol or a mixture of water and polyethylene glycol as the non-lipid liquid. Specifically, I have found that polyethylene glycol is particularly useful, since it is approved as a pharmaceutical component, it has the desired viscosity and surface tension for incorporation into the final emulsion, and it passes unaltered through the digestive system. Polyethylene glycol 400 (PEG 400) is $H(OCH_2CH_2)_nOH$, wherein n is from 8.2 to 9.1 and the molecular weight is from about 380 to about 420. It is a viscous, slightly hydroscopic liquid and it has a viscosity of 7.2 Centistokes at 210°F. Of course, other polyethylene glycols ranging in molecular weight from about 200 to about 9,000 are available and can be utilized, if appropriate.

Preferably, the non-lipid liquid is selected from the group consisting of polyethylene glycol or mixtures of polyethylene glycol and water. Preferably, the water constitutes 50% or less of the glycol-water mixture. Where the emulsion is to be incorporated into a soft gelatin capsule, the water constitutes 20% or less of the total emulsion, and where the emulsion is to be incorporated into a hard gelatin capsule, the water constitutes about 10% or less of the total emulsion.

As an aid in forming the emulsion and specifically to increase the surface tension of the layer of non-lipid liquid which surrounds each of the lipid globules, fumed silica can be added to the mixture prior to emulsification.

Fumed silica is a colloidal form of silica made by the combustion of silicon tetrachloride in a hydrogen-oxygen furnace. It is an extremely fine white powder having an extremely large exposed surface area. The utilization of the fumed silica seems to thin out or reduce the thickness of the enveloping non-lipid layer upon each of the lipid globules, and the fumed silica also improves the stability of the final emulsion.

The formation of the emulsion can be effected by any desired technique as well known in the art. Satisfactory emulsions have been formed by simply utilizing a hand-held mixer operated at the highest speed. However, such emulsions are of limited fineness. Finer emulsions can be obtained by utilizing commercially available emulsifying and homogenizing equipment, such as Gaulin emulsifiers or homogenizers, which are capable of operating at high pressures, typically on the order of 8-9,000 psi. The emulsion ingredients can be passed through a Gaulin homogenizer several times to obtain emulsions of increasing fineness and of increased stability.

-14-

Ultrasonic vibration equipment can also be utilized to both form the emulsion of requisite globular size and to micronize the drug to ensure its incorporation into the lipid ingredient of the emulsion. Generally such equipment utilizes a rod which is inserted into the liquid and vibrated sonically at an extremely high frequency. Where the drug is non fully lipid-soluble, the drug, in either solid or liquid form, is mixed with the lipid or mixed with one or more surfactants and the mixture is vibrated ultrasonically to reduce the drug to extremely small or micronized solid particles or liquid droplets within the lipid or surfactants. The remainder of the emulsion ingredients are then added, and the mixture is again vibrated ultrasonically to reduce the lipid to its final globular size with the drug contained within each globule and forming the non-lipid liquid coating encasing each lipid globule. By the utilization of such ultrasonic equipment, it has been found that lipid globule sizes in the range of about 0.1 to about 0.5 microns can be readily obtained, and even finer globules are possible.

The order of ingredient addition to form the emulsion may be varied, primarily in accordance with the pharmaceutical. Where the drug is lipid-soluble, it is merely dissolved in the lipid, as a first step, and the surfactant is used to form the emulsion. Where the drug is lipid-insoluble and not highly ionic, the drug is added to the lipid and dispersed therein by the surfactant which can be added to the lipid either with or before the drug is added. In some cases, such as with insulin, the drug preferably is initially wetted with the lipid prior to addition of the surfactant. Where the drug is highly ionic, the drug may be added to the surfactant or surfactant mixture before the resulting mixture is added to the lipid. This is preferred, for example, in formulating atenolol emulsions.

-15-

The final emulsion thus comprises a myriad of lipid globules of a size smaller than about 3 microns and preferably predominantly 1 micron or less in size, each globule containing the pharmaceutical or drug dispersed therein, either in solution or in finely-divided suspended form or, in the case of partially lipid-soluble drugs, in both solution and suspension. Each lipid globule is surrounded by a layer of non-lipid liquid capable of passing through the digestive system without alteration. This non-lipid layer may, of course, contain very minor amounts of the surfactant components and the fumed silica components of the pre-emulsion mixture and, in the event that the pharmaceutical or drug is soluble in the non-lipid liquid, some of the drug component as well. This enveloping layer preferably is polyethylene glycol or a mixture of polyethylene glycol and about 30% or less water.

The final emulsion can be packaged in a hard gelatin capsule or in a soft gelatin capsule or, if desired, can be administered as a liquid, in which case the amount of water in the emulsion is immaterial.

In one specific case, where the pharmaceutical ingredient was atenolol and the soft capsule was gelatin containing glycerin as a plasticizer, some of the glycerin plasticizer migrated into the capsule contents and entered the emulsion prior to drying. On drying of the capsule, the glycerin retreated into the capsule shell, taking some of the emulsion with it. It is believed that the emulsion was not broken, and that the atenolol remained in its dispersed form in the lipid globules even in the capsule shell. However, in such instances and particularly with highly ioninc or polar drugs, the problem can be avoided by eliminating glycerin from the capsule ingredients. Alternate plasticizers, such as sorbitol or polyethylene glycol can be utilized and are preferred.

As above explained, the emulsion can contain up to 20% water where it is to be encapsulated in soft shell capsules.

As above explained, the emulsions of the present invention can be encapsulated into hard gelatin capsules, so long as (a) the emulsion contains no more than about 10% water, preferably the non-lipid ingredient is polyethylene glycol and an amount of water ranging from about 0 to about 10% of the total emulsion ingredients, and (b) the capsule is sealed by any desired technique. The capsule can be sealed by an appropriate, commercially available banding technique supplied by Eli Lilly, Inc. of Indianapolis, Indiana, U.S.A., or by the utilization of the compositions and techniques disclosed in my co-pending United States Application Serial No. 856,348, filed on April 28, 1986.

Of course, the water content of the emulsions is limited to prevent dissolution or softening of the gelatin of the hard capsule. Generally, it is possible to make satisfactory products encapsulated in hard capsules when the water content of the encapsulated emulsion mixture is about 10% or less. If the resultant filled capsule is excessively soft, this problem can be cured by reducing or eliminating the water content, preferably by the use of polyethylene glycol as a water substitute.

## EXAMPLE I

A composition was prepared containing:

| Ingredient | Weight in Grams |
|---|---|
| Corn Oil | 100 |
| PEG 400 | 76 |
| Tween 20 | 16 |

| | |
|---|---|
| Tween 80 | 14 |
| Span 80 | 23 |
| Fumed Silica | 4 |
| Water | 10 |
| Atenolol | 25 |

The mixing procedure was as follows:

1. The surfactants were mixed.

2. The atenolol was dissolved in the surfactant mixture.

3. The corn oil was then added and mixed.

4. The fumed silica, PEG 400 and water were then added and mixed.

5. The final mixture was then passed several times through the Gaulin homogenizer at a pressure of 8000 psi.

Microscopic examination of the emulsion showed that 50% of the oil globules were about 0.5 microns in size or smaller, and virtually all of the oil globules were less than about 1 micron in size. The atenolol was dispersed in the oil globules which were surrounded by an enveloping layer of polyethylene glycol plus water with some surfactant.

The emulsion remained stable for several days, and it passed the Food and Drug Administration stability test indicating a shelf life of six months.

Some of the emulsion was packaged in soft gelatin capsules, each capsule containing 25 milligrams of atenolol.

## EXAMPLE II

A composition was prepared containing:

| Ingredient | Weight in Grams |
|---|---|
| Corn Oil | 109 |
| PEG 400 | 65.4 |
| Tween 20 | 27 |

0212875

| | |
|---|---|
| Tween 80 | 22 |
| Span 80 | 16.4 |
| Fumed Silica | 4.3 |
| Hydrocortisone | 0.150 |

The mixing procedure was as follows:

1. The hydrocortisone was dissolved in the corn oil.

2. The surfactants were added.

3. The PEG 400 was added.

4. The fumed silica was added.

5. The emulsion readily formed on mixing with a hand mixer, and continued mixing improved the fineness of the emulsion.

The emulsion remained stable for 2 days, and no separation was noted upon microscopic examination. The total emulsion was sufficient to make about 1,000 capsules of 25 mg weight each. However, only 10 capsules were filled and stored.

### EXAMPLE III

A composition was prepared containing:

| Ingredient | Weight in Grams |
|---|---|
| Corn Oil | 96 |
| PEG 400 | 57.6 |
| Tween 20 | 24 |
| Tween 80 | 19.2 |
| Span 80 | 14.4 |
| Fumed Silica | 3.8 |
| Water | 24 |
| Hydrocortisone | 0.150 |

The mixing procedure (with the exception of the water addition) and the results were as described in Example II.

## EXAMPLE IV

A composition was prepared as in Example I, but with 0.147 grams of insulin (27.1 units of insulin per mg of insulin) substituted for the hydrocortisone, and insulin being dissolved in the mixture of surfactants. The relative amounts of some of the ingredients were changed, but the same mixing and emulsifying procedure was followed.

Sixty soft gelatin capsules containing about 250 mg of emulsion were prepared. Each capsule contained:

| Ingredient | Weight in mg |
|---|---|
| Corn Oil | 96.000 |
| PEG 400 | 57.600 |
| Tween 20 | 24.000 |
| Tween 80 | 19.200 |
| Span 80 | 14.400 |
| Fumed Silica | 3.840 |
| Insulin (27.1 units/mg of insulin) | 0.147 (4 insulin units) |
| Water | 24.000 |

The final emulsion was stable on storage and had an oil globule size of about 1 micron.

## EXAMPLE V

A composition was prepared using the procedure of Example II, but the relative amounts of the ingredients were changed to yield the following composition containing insulin instead of hydrocortisone.

Sixty capsules were prepared, each capsule containing:

| Ingredient | Weight in Milligrams |
|---|---|
| Insulin (27.1 units/mg of insulin) | 0.147 or 4 insulin units |
| Corn Oil | 109.000 |
| Tween 20 | 23.200 |
| Tween 80 | 21.800 |
| Span 80 | 16.350 |

0212875

| | |
|---|---|
| PEG 400 | 65.400 |
| Fumed Silica | 4.300 |

The emulsion, upon microscopic examination, showed the presence of oil globules about 1 micron in size. The emulsion was stable upon storage.

## EXAMPLE VI

Three diabetic dogs were each tested on five (5) days. Each dog was a Type I diabetic and had been maintained since diagnosis on injections of insulin. The dogs currently were maintained on NPH insulin (a long-acting form of injectible insulin) injections twice day at 0900 hours and at 1600 hours.

The current maintenance doses of the dogs are as follows:

Dog I (Ruffian) is a female, born January 1979 and diagnosed as diabetic during February 1979. Her normal insulin control is 17 units of NPH insulin injected at 0900, and 5 units of NPH insulin injected at 1600 hours on a daily basis.

Dog II (Morgan) is a female, born September 1981 and diagnosed in December 1981. Her normal control is an injection of 12 units of NPH insulin and 5 units of regular insulin at 0900 and an injection of 18 units of NPH insulin at 1600 hours.

Dog III (Buzz) is a male, born November 1978 and diagnosed as diabetic in February of 1979. He receives 20 units of NPH insulin at 0900 hours and 24 units of NPH insulin at 1600, both by injection.

Dog I (Ruffian) came into heat part-way through testing. This condition causes an increase in temperature and more difficult diabetic control. Further, the generalized edema probably decreased absorption control from the G.I. tract.

The dogs were each maintained on their normal schedule on November 4, 1985, receiving their injections at 0900 and 1800 hours and being fed at 0900 hours. On November 5, November 6, November 11 and November 15, the dogs were given the indicated dosages of oral insulin capsules at 0900, plus a saline injection to avoid altering the psychological controls of the dogs. The insulin capsules were given in one or more meatballs during the normal feeding time, and they were given their regular insulin injections at 1600. The dogs maintained their normal activity during each day. During the periods November 6 to November 11 and November 11 to November 15, the dogs resumed their normal two injection daily routine.

The blood sugar levels of each dog were determined every hour and recorded as hereafter reported. The determinations were made by the use of Glucocheck II strips which are effective and accurate at blood sugar levels of 450 or less.

The results of the dog tests are reported as follows:

### DOG I (RUFFIAN)

| TIME OF DAY | 11/4/85 (Control) | 11/5/85 | 11/6/85 | 11/11/85 | 11/15/85 |
|---|---|---|---|---|---|
| **DOSAGE** | | | | | |
| 0900 Hrs. | 17 units, NPH insulin by injection | 8 units oral insulin of Ex. IV | 16 units oral insulin of Ex. IV | 28 units oral insulin of Ex. IV | 56 units insulin of Ex. V |
| 1600 Hrs. | 5 units, NPH insulin by injection | 5 units, NPH injection | 5 units, NPH injection | 5 units, NPH injection | 5 units, NPH injection |
| | BS (1) | BS | BS | BS | BS |
| 0800 | Hi (2) | Hi | 288 | 63 | 212 |
| 0900 | Hi (FEED) | 306 (FEED) | 218 (FEED) | 68 (FEED) | 311 (FEED) |
| 1000 | 319 | 275 | 272 | 79 | 362 |
| 1100 | 62 | 310 | Hi | 185 | 350 |
| 1200 | 74 | 400 | Hi | 200 | 412 |
| 1300 | 94 | Hi | Hi | 211 | 311 |

| 1400 | 79 | Hi | -- | -- | 412 |
|---|---|---|---|---|---|
| 1500 | 102 | Hi | -- | -- | -- |
| 1600 | 95 | -- | -- | -- | -- |
| 1700 | 55 | -- | -- | -- | -- |

On November 4, 1985 and November 5, 1985, Dog I (Ruffian) came into heat.

(1) BS means Blood Sugar Level

(2) Hi means in excess of about 450

## DOG II (MORGAN)

| TIME OF DAY | 11/4/85 (Control) | 11/5/85 | 11/6/85 | 11/11/85 | 11/15/85 |
|---|---|---|---|---|---|
| **DOSAGE:** | | | | | |
| 0900 Hrs. | 12 units, NPH insulin 5 units reg. insulin by injection | 8 units oral insulin of Ex. IV | 16 units oral insulin of Ex. IV | 28 units oral insulin of Ex. IV | 56 units oral insulin of Ex IV |
| 1600 Hrs. | 18 units NPH insulin by injection | 18 units, NPH insulin by injection | 18 units, NPH insulin by injection | 18 units, NPH insulin by injection | 18 units NPH insulin by injection |
| | BS (1) | BS | BS | BS | BS |
| 0800 | 117 | 157 | Hi | 71 | Hi |
| 0900 | 67 (FEED) | 237 (FEED) | Hi (FEED) | 146 (FEED) | Hi (FEED) |
| 1000 | 69 | 219 | 387 | 149 | 401 |
| 1100 | 53 | 276 | Hi | 354 | 376 |
| 1200 | 55 | 371 | Hi | 386 | 362 |
| 1300 | 58 | Hi (2) | Hi | 399 | 400 |
| 1400 | 114 | Hi | -- | -- | 400 |
| 1500 | 87 | 319 | -- | -- | -- |
| 1600 | 109 | -- | -- | -- | -- |
| 1700 | 54 | -- | -- | -- | -- |

0212875

## DOG III (BUZZ)

| TIME OF DAY | 11/4/85 (CONTROL) | 11/5/85 | 11/6/85 | 11/11/85 | 11/15/85 |
|---|---|---|---|---|---|
| **DOSAGE:** | | | | | |
| 0900 Hrs. | 20 units, NPH insulin injection | 12 units oral insulin, Ex. V | 20 units oral insulin, Ex. V | 32 units oral insulin, Ex. V | 64 units oral insulin, Ex. V |
| 1600 Hrs. | 24 units, NPH insulin injection | 24 units, NPH insulin injection | 24 units, NPH insulin injection | 24 units NPH insulin injection | 24 units, NPH in-sulin by injection |
| | BS (1) | BS | BS | BS | BS |
| 0800 | 63 | 59 | 210 | 385 | 150 |
| 0900 | 63 (FEED) | 154 (FEED) | 272 (FEED) | 261 (FEED) | 376· (FEED) |
| 1000 | 135 | 233 | Hi | 345 | 368 |
| 1100 | 72 | 258 | Hi | Hi | 372 |
| 1200 | 53 | 403 | 383 | Hi | 400 |
| 1300 | 85 | 275 | Hi | Hi | 341 |
| 1400 | 105 | Hi (2) | -- | -- | 349 |
| 1500 | 75 | Hi | -- | -- | -- |
| 1600 | 67 | -- | -- | -- | -- |
| 1700 | 61 | -- | -- | -- | -- |

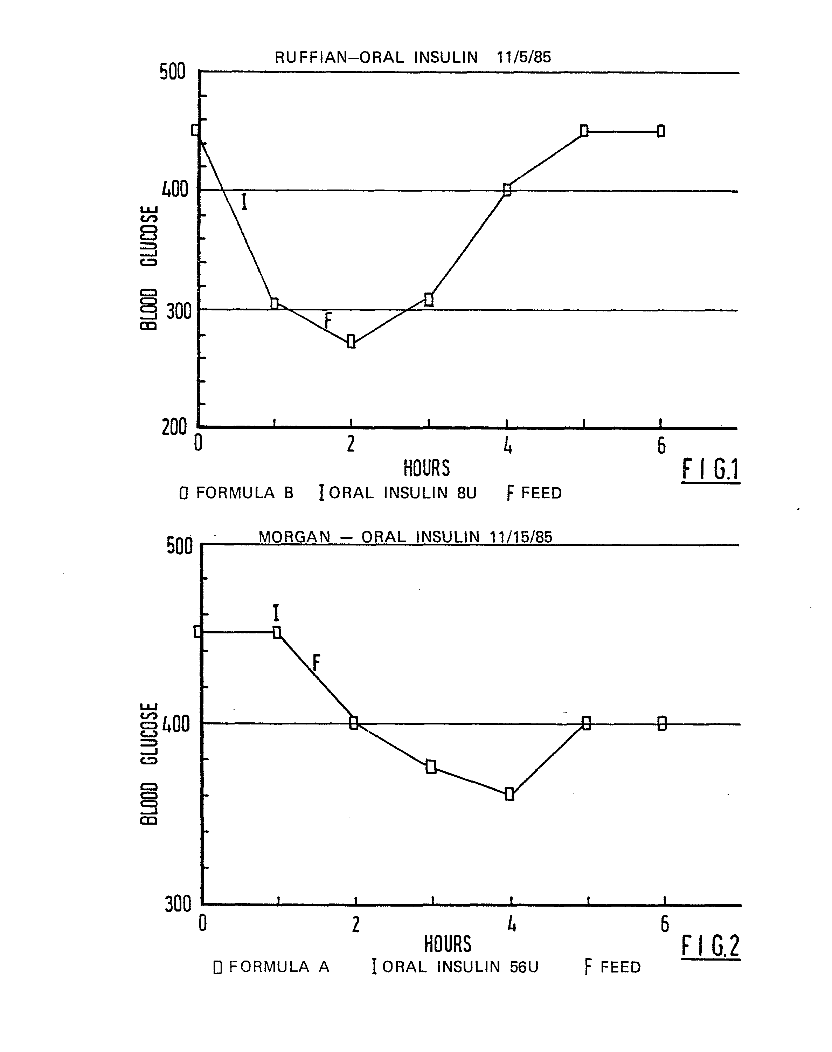

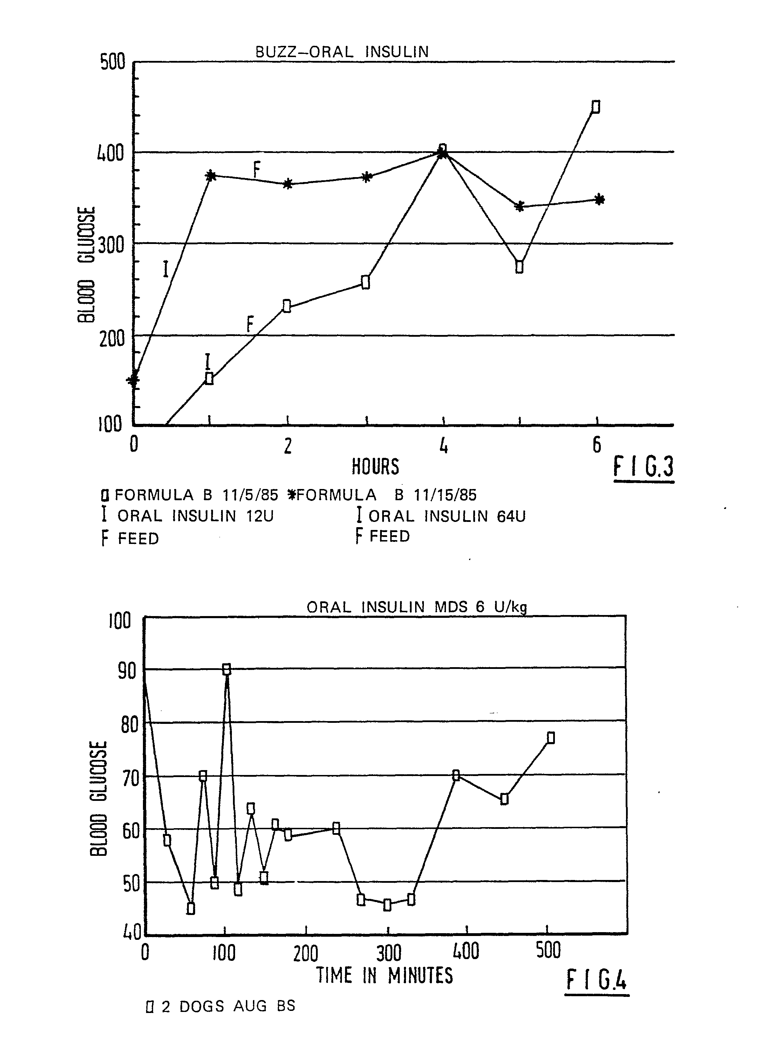

These test results are graphically illustrated in Figures 1-3.

Discussion:

From the test results, it appears that both the formulation of Example IV and Example V are effective to deliver orally-injected insulin to the bloodstream of the dogs. The effect of the Example V formulation in reducing blood sugar level appeared to be greater than that of Example IV.

Dog III (Buzz) is a severely diabetic animal, and he rapidly loses insulin control. Yet the test of November 15, 1985 achieved some percentage of insulin absorption, probably on the order of 30 to 40%.

The result with Dog I (Ruffian) and Dog II (Morgan) clearly indicate insulin absorption from the orally administered capsules.

### EXAMPLE VII

An emulsion was prepared of essentially the same formulation as that of Example V, but with the substitution of doxorubicin (Adriamycin) for the insulin of Example V, and the substitution of sesame seed oil for the corn oil. The doxorubicin was suspended as a solid in the sesame seed oil by stirring, then the PEG 400 was added, then the surfactants and the fumed silica. The emulsion was prepared by the use of a hand mixer, and examination indicated an oil globule size predominantly on the order of about 3 microns or less. The emulsion was stable on storage.

Each dosage unit of the emulsion, if incorporated into capsules capable of delivering 2 mg of doxorubicin, would contain:

| Ingredient | Weight in Milligrams |
|---|---|
| Doxorubicin | 2.000 |
| Sesame Seed Oil | 109.000 |
| Tween 20 | 23.200 |
| Tween 80 | 21.800 |
| Span 80 | 16.350 |
| PEG 400 | 65.400 |
| Fumed Silica | 4.3 |

### EXAMPLE VIII

83.23 grams of gum arabic and 166.68 grams of sterile water were mixed, utilizing a magnetic mixer. 33.25 grams of sesame seed oil and 24.30 grams of propranolol HCl were mixed in an ultrasonic mixer to suspend the propranolol in the sesame seed oil. The propranolol-sesame seed oil suspension was then added to the gum arabic-water mixture and blended utilizing a laboratory propeller blender or micronizer.

This mixture was then processed in a Gaulin 15M homogenizer at 8,000 pounds per square inch first pass. Microscopic examination showed that most of the emulsion contained oil globules less than 3.0 microns in size. The emulsion was then passed a second time through the Gaulin homogenizer at 8,000 p.s.i., and microscopic examination showed that 90% of the emulsion contained sesame seed oil globules less than 1 micron in diameter. However, the viscosity of the emulsion was too great to load soft gelatin capsules.

An additional 229.90 grams of sterile water was added to the emulsion to reduce its viscosity. 500 grams of the emulsion was segregated and 220 grams of sesame seed oil and 240 grams of PEG 400 was added. The mixture of emulsion containing the additional sesame seed oil and the PEG 400 was passed again through the homogenizer. The resultant emulsion was of a low enough viscosity to flow for the filling of soft gelatins.

1,900 capsules were filled and the capsules were dried for 48 hours. Each capsule contained 10 mg of propranolol. Because of the high water content, the formed capsules were indented, but 1,300 of the capsules had minimal indentations and were suitable for use. Upon examination the emulsion in the capsules was determined to consist of oil globules, 90% of the globules being of a size less than 1 micron.

Three healthy adult patients were each given one 10 mg of commercially available propranolol HCl capsules on Day 1 as a control. The blood serum level of each patient was then determined by the Toxicology Center in Lansing, Michigan, an institution licensed by the Center for Disease Control. The blood serum level was determined by the use of HPLC, i.e., a high performance liquid chromotography apparatus, after the lapse of 1, 2, 3, 4 and 6 hours, respectively.

On Day 4, the same three patients were given capsules prepared as in this Example VIII, also containing a total of 10 mg of propranolol HCl, and the blood serum level of each patient was determined as above set forth.

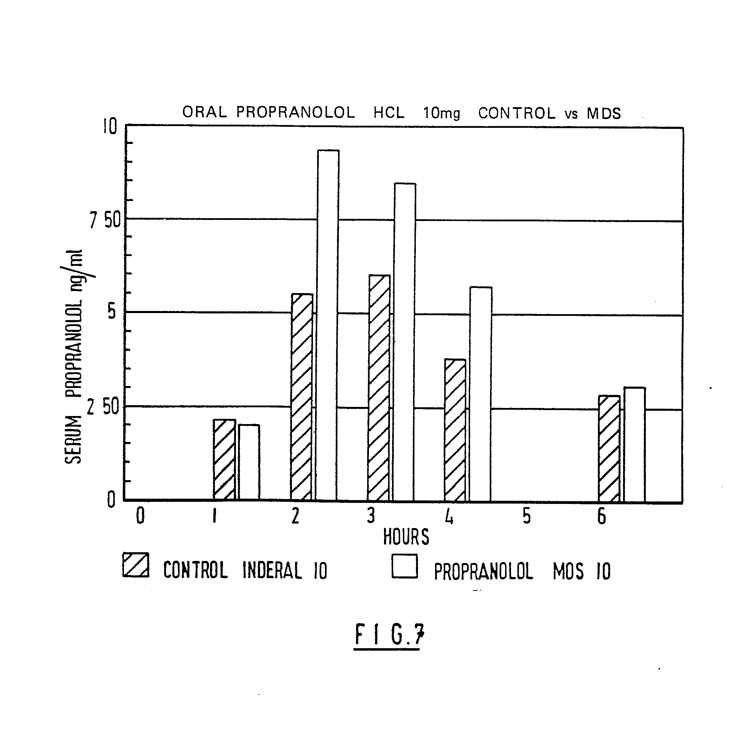

The following results were obtained, as shown in Figure 7:

|  | BLOOD SERUM LEVEL IN NANOGRAMS PER MILLILITER | | |
|---|---|---|---|
|  | CONTROL | MICROL DELIVERY | % INCREASE |
| 1 Hour | 2.167 | 2.0 | -- |
| 2 Hours | 5.5 | 9.389 | 64.3 |
| 3 Hours | 6.067 | 8.50 | 52.9 |
| 4 Hours | 3.833 | 5.722 | 52.3 |
| 6 Hours | 2.867 | 3.112 | 12.7 |

To avoid the presence of excess water, and to prevent interference with the composition by the gum arabic, the following preferred formulation can be utilized:

| Tween 80 | 100 grams |
|---|---|
| Sterile Water | 100 grams |
| PEG 400 | 100 grams |
| Sesame Seed Oil | 400 grams |
| Propranolol HCl | 28 grams |

A 260 milligram capsule will contain 10 milligrams of propranolol.

### EXAMPLE IX

Following the same procedure as outlined in Example VIII, an emulsion containing 83.24 grams of gum arabic; 166.49 grams of sterile water; 333.02 grams of sesame seed oil; and 64.75 grams of chlorthalidone was prepared.

Difficulty was encountered in passing the emulsion through the Gaulin homogenizer at 8,000 p.s.i., because the homogenizer valve hangs up with the large particle mass of the chlorthalidone. An additional 846.49 grams of water was added to the emulsion and it was passed through the homogenizer at 8,000 p.s.i. for a second time. Particle size analysis showed that 90% of the sesame seed oil globules were less than 1 micron in diameter.

Next, 200 grams of Tween 80 and 220 grams of PEG 400 was added to the emulsion, and it was passed through the homogenizer at 8,000 p.s.i. again. The final emulsion was encapsulated in soft gelatin capsules. The capsules were dried for 48 hours and stored. No water drying effect or indentations were found in the capsules.

It is desirable to vigorously break up the non-lipid soluble pharmaceutical in its dispersion in the oil prior to forming the emulsion. This can be done by passing the pharmaceutical-lipid mixture through a micronizer such as a "Dyno-mill." A preferred formulation for chlorthalidone would be as follows:

| | |
|---|---|
| Tween 80 | 100 grams |
| Sterile Water | 100 grams |
| PEG 400 | 100 grams |
| Sesame Seed Oil | 400 grams |
| Chlorthalidone | 70 grams |

This emulsion will yield 2800 soft gelatin capsules, each containing 25 milligrams of chlorthalidone.

### EXAMPLE X

333.73 grams of sesame seed oil and 146.00 grams of triamterene were mixed utilizing an ultrasonic mixer. The triamterene was more soluble in the sesame seed oil than were the pharmaceuticals of Examples VIII and IX. 84.42 grams of gum arabic and 83.80 grams of sterile water were mixed separately utilizing a magnetic mixer and were added to the oil-triamterene dispersion and mixed utilizing a laboratory propellor mixer. The resultant emulsion was passed through a Gaulin homogenizer at 8,000 p.s.i., and a first pass gave an emulsion size in which 90% of the globules were less than 1 micron in diameter. To reduce the water content, 200 grams of Tween 80 and 220 grams of PEG 400 were added to the emulsion and it was again passed through the homogenizer at 8,000 p.s.i. 3,480 capsules were prepared by soft gelatin encapsulation, and the capsules were dried for 72 hours. Each capsule contained 25 milligrams of triamterene.

A more preferred formulation for making 2,800 capsules, each of 300 milligrams and containing 50 milligrams of triamterene could be made from the following:

| | |
|---|---|
| Tween 80 | 100 grams |
| Sterile Water | 100 grams |
| PEG 400 | 100 grams |
| Sesame Seed Oil | 400 grams |
| Triamterene | 140 grams |

## EXAMPLE XI

An emulsion was prepared in accordance with the procedure of Example XIII containing the following ingredients:

| | |
|---|---|
| Gum Arabic | 83.24 grams |
| Sterile Water | 166.49 grams |
| Sesame Seed Oil | 333.02 grams |
| Hydrochlorathiazide | 64.75 grams |

Upon mixture of the ingredients, the emulsion separated because the negative charge of the gum arabic caused dissociation of the emulsion when mixed with the negatively charged hydrochlorathiazide.

An acceptable formulation would be as follows:

| | |
|---|---|
| Tween 80 | 100 grams |
| Sterile Water | 100 grams |
| PEG 400 | 100 grams |
| Sesame Seed Oil | 400 grams |
| Hydrochlorathiazide | 64.75 grams |

In addition to changing the formulation, the pharmaceutical would be micronized with the oil to disperse the drug in the oil, as in a Dyno-mill, prior to adding the Tween, water and PEG 400 ingredients and homogenizing to form the final emulsion.

## EXAMPLE XII

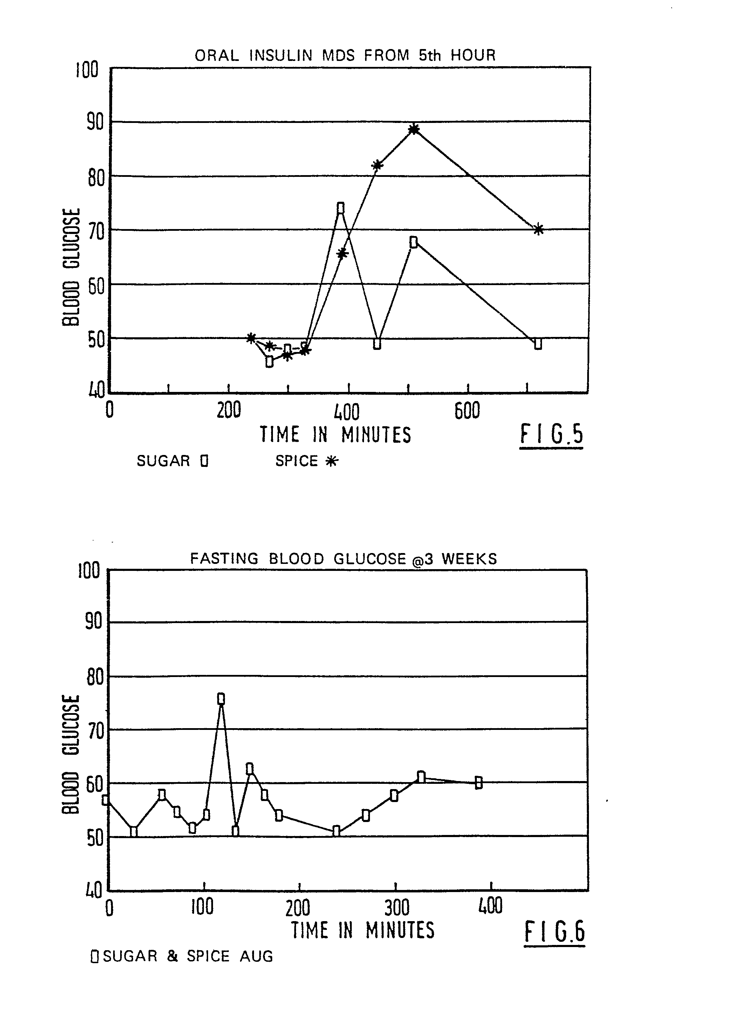

Sugar and Spice are two healthy, normal, non-diabetic female beagles. The animals were fasting from 10 A.M. on the day prior to the start of the experiment. Oral insulin in the formulation of the prior Example VI at a dosage of 6 insulin units per kilogram of the respective dog's weight was administered at 0 hours (8 A.M.) on the experimental day. Samples of each dog's blood were taken and the blood glucose levels were determined at the intervals indicated in Figure 4.

At 60 minutes, they reached their maximum hypoglycemic effect and during the next 60 minutes there were two elevated spikes in their blood sugar. These spikes were interpreted to represent the counter regulatory hormones effect and the non-glucose substrates effect, as recognized in the literature at Cardiovascular Reviews & Reports, Vol. 7, No. 7, July 1986 at pages 649-656.. Over the next 150 minutes, they maintained minimal hypoglycemic effect. At 270 minutes they returned to the 40 range which was maintained over the following 60 minutes.

The animals were fed at 450 minutes. The elevation of their blood glucose followed feeding. The chart of Figure 4 indicates the average blood glucose on both dogs through 500 minutes.

After feeding, more normal elevation occurred with Spice; Sugar did not achieve a significant response to the food. Detail of blood glucose on each dog is set out on the chart of Figure 5, but note that actual levels on the first 300 minutes is lost in the longer time frame.

Three weeks later, a fasting blood sugar level without insulin was obtained on both dogs, as shown in Figure 6.

In this test three weeks later and to the surprise of the investigators, both dogs were running at blood sugar levels in the 50's throughout the day with the exception of the spike at 10 A.M. which is the normal time for the dogs to be fed. The spike, at 80, was not as high as it was the first day. The dogs then returned to a level averaging 57 for the remainder of the experimental day. One week later, the animals were again tested in the morning and they were back to a normal range of blood sugar level.

0212875

In attempting to understand this long-term result, it is postulated that by delivering the insulin in the lipid globule intact through the lymphatic system, the phagocytic cells of the reticuloendethial system are capturing these foreign body globules and creating a slow release over time. Also, prevention of normal metabolism of the insulin would occur under these circumstances. This theory appears to be consistent with the results of the tests, particularly the test conducted three weeks after the only administration of insulin.

## CLAIMS

1. A medicinal composition comprising lipid, non-lipid liquid and pharmaceutical ingredients, characterized by the fact that the ingredients are emulsified and encapsulated in a gelatin capsule as a liquid and in a physical form capable of delivering the pharmaceutical in its unaltered original state to the brush layer of the small intestine; the non-lipid ingredient being water, a water substitute not attacked by lipid enzyme activity or a mixture of water substitute and water; and the total water in the emulsion is not more than 20%; the physical form of the ingredients is as follows:

    (a) the lipid ingredient is in the form of individual globules predominantly of a size less than about 3 microns in diameter;

    (b) each of the globules contains the pharmaceutical ingredient dispersed therein; and

    (c) each of the globules is enveloped within a surrounding layer of said non-lipid liquid ingredient adhered to the globule by surface tension, the surrounding layer serving to maintain the globules in independent form and to present only the non-lipid liquid surface to the stomach fluids.

2. A composition as claimed in Claim 1, further characterized by the composition being encapsulated in a hard gelatin capsule, and the total water in the emulsion being not more than about 10%.

3. A composition as claimed in Claims 1 or 2, further characterized by the water substitute being polyethylene glycol.

4. A composition as claimed in Claims 1 or 2, further characterized by the non-lipid liquid being polyethylene glycol and from 0 to about 50% water.

5. A composition as claimed in Claim 1, further characterized by the composition containing at least one surfactant.

0212875

6.  A composition as claimed in Claims 1 through 5, further characterized by the lipid globules being predominantly of a size of 1 micron or less in diameter.

7.  A composition as claimed in Claims 1 through 6, further characterized by the pharmaceutical ingredient being insulin.

8.  A composition as claimed in Claims 1 through 6, further characterized by the pharmaceutical ingredient being atenolol.

9.  A composition as claimed in Claims 1 through 6, further characterized by the pharmaceutical ingredient being doxorubicin.

10.  A method of making a medicinal composition as claimed in Claim 1 through 9, comprising the steps of:

(a)  dispersing a pharmaceutical in a lipid;

(b)  forming an emulsion comprising the lipid having the pharmacutical dispersed therein, a non-lipid liquid not attacked by lipid enzyme activity, and at least one surfactant, the emulsion containing not more than 20% total water;

(c)  homogenizing the emulsion to reduce the lipid to individual pharmaceutical-containing globules having a diameter predominantly less than 3 microns in diameter, and each globule being enveloped in a surrounding layer of the non-lipid liquid; and

(d)  encapsulating the emulsion in a gelatin capsule.

11.  The method as claimed in Claim 10, further characterized by the pharmaceutical first being wetted by the oil prior to forming the emulsion.

12.  The method as claimed in Claims 10 or 12, further characterized by mixing the pharmaceutical with at least one surfactant prior to forming the emulsion which is the same surfactant utilized to form the emulsion.

13.  The method as claimed in Claims 10 through 12, further characterized by the addition of fumed silica to the emulsion ingredients.

RUFFIAN—ORAL INSULIN   11/5/85

BLOOD GLUCOSE (y-axis: 200, 300, 400, 500)
HOURS (x-axis: 0, 2, 4, 6)

□ FORMULA B    I ORAL INSULIN 8U    F FEED

**F I G.1**

MORGAN — ORAL INSULIN 11/15/85

BLOOD GLUCOSE (y-axis: 300, 400, 500)
HOURS (x-axis: 0, 2, 4, 6)

□ FORMULA A    I ORAL INSULIN 56U    F FEED

**F I G.2**

BUZZ—ORAL INSULIN

□ FORMULA B 11/5/85  ＊FORMULA  B 11/15/85
I ORAL INSULIN 12U        I ORAL INSULIN 64U
F FEED                    F FEED

FIG.3

ORAL INSULIN MDS 6 U/kg

TIME IN MINUTES

FIG.4

□ 2 DOGS AUG BS

0212875

ORAL INSULIN MDS FROM 5th HOUR

FIG.5

SUGAR ▢    SPICE ✳

FASTING BLOOD GLUCOSE @3 WEEKS

FIG.6

▢ SUGAR & SPICE AUG

ORAL PROPRANOLOL HCL 10mg CONTROL vs MDS

SERUM PROPRANOLOL ng/ml

HOURS

▨ CONTROL INDERAL 10   ☐ PROPRANOLOL MOS 10

F I G.7